# EUROPEAN PATENT APPLICATION

(11) **EP 1 724 579 A2**
(43) Date of publication of application: **22.11.2006**
(21) Application number: 06010523.6
(22) Date of filing: 22.05.2006
(51) Int. Cl.: G01N 33/50

(54) **Methods of prostate cancer diagnosis and prostate tumour analysis**

(30) Priority: 20.05.2005 GB 0510352
(71) Applicant: Optomed as, 7044 Trondheim (NO)
(72) Inventor: Ellingsen, Reinhold, 7989 Heimdal (NO); Hjelme, Dag Roar, 7030 Trondheim (NO); Östling, Dan, 7030 Trondheim (NO); Waehre, Hakon, 1395 Hvalstad (NO); Paus, Elisabeth, 0776 Oslo (NO); Giercksky, Karl-Erik, 1362 Hosle (NO)
(74) Representative: Gardner, Rebecca Katherine

(57) **Abstract**

The invention relates to a method of diagnosing prostate cancer in a subject, which method comprises determining the concentration of a relevant biochemical marker in the periprostatic tissue or lymph nodes of said subject, also to methods which categorise a prostate tumour and/or identify a prostate tumour which has extended beyond the prostatic capsule; as well as to apparatus which is suitable for use in such methods.

## Description

The present invention relates to methods of obtaining clinically relevant information about solid tumours, in particular tumours of the prostate.

Prostate cancer is a common disease in men above 50 years. Prostate cancer is the most frequent type of cancer in males - accounting for 25% of all cancers in the male population. However, suitable treatment and management of prostrate cancer is not simply a matter of identifying all those men who have an invasive cancer and commencing therapy, as the following figures discussed by Patrick C. Walsh M.D. US Urologist-in-Chief, demonstrate. In autopsies on men over 50 years of age, microscopic examination of the prostate gland demonstrates the presence of invasive cancer in 40% of subjects. This surprisingly high incidence of invasive histologic cancer occurs in men of all nationalities and increases with advancing age. In contrast, however, based on the National Cancer Institute's annual SEER surveys (Surveillance, Epidemiology and End Results), only 8% of men in the U.S. will present with clinically significant disease in their lifetime affecting their quality of life. Furthermore, only 3% of all men in the U.S. die of prostate cancer. In no other human cancer is there such an enormous disparity between the very high incidence of malignancy microscopically and relatively low death rate. The slow growth rate of most prostate cancers in part explains the above figures.

Current diagnostic methods include rectal examinations, prostate biopsies and analysis of systemic blood samples for prostate specific antigen (PSA), a glycoprotein enzyme produced exclusively by prostatic epithelium. However rectal examinations and biopsies are rather unpleasant and invasive procedures which can easily miss small tumours. In addition, some prostate tumours are much more aggressive than others and so a small tumour will sometimes grow to a threatening size in a timescale incompatible with a normal testing regimen.

Over reliance on serum PSA levels can be problematic due to the effect on those levels of Benign prostatic hyperplasia (BPH). Benign prostatic hyperplasia - the benign growth of prostatic tissue that most men develop over the age of 50 - also increases serum PSA (albeit one-tenth as much as the same volume of cancer). Some men grow very large amounts of BPH. In those with over 50cm³ of BPH, the total amount of PSA produced can cause such an elevation of serum PSA that it may mask the rise in PSA caused by cancer, especially a smaller cancer of 1 to 2cm³. Complicating the problem imposed by very large benign prostates is that in a small subset of men with very large prostates, the BPH tissue grows so rapidly that it can approach the doubling time of PSA in prostate cancer.

At present there are no diagnostic tools with high sensitivity and specificity for determining a tumour's extension, i.e. whether it is intracapsular or extracapsular. Digital rectal exploration and Trans Rectal Ultra-Sound guided biopsies often result in underestimation of the tumour extension, while magnetic resonance imaging (MRI) has a tendency to overestimate tumour extension.

Serious illness, impairment of quality of life and death are rarely due to prostate cancer which is fully defined within the organ itself. Instead these occur when the tumour escapes the confines of the prostate capsule and is then able to metastasise and spread to the lymph nodes. The term prostate 'capsule' is conveniently used to define the boundary of the prostate and refers to a layer of condensed smooth muscle and connective tissue of variable thickness. It blends with the prostatic sheath and with part of the Denonvilliers' fascia and in parts thins such that on many surgical specimens no capsule as such is found in these areas at all.

Radical prostatectomy is considered to be an adequate treatment for patients with organ defined prostate cancer. However, common practice has shown that about 1/3 of the patients undergoing radical prostatectomy would have fared better with alternative treatments (e.g. hormone or radiation therapies) to tumour growth external to the prostate capsule.

In attempting to improve on existing methods of classification and diagnosis of prostate cancer the inventors have developed new methods of prostate cancer diagnosis based on the measurement of the concentration of biochemical markers (such as PSA) which are associated with the prostate and prostate cancer.
Unlike previous PSA assays the present invention does not involve measurement of systemic (i.e. blood serum) PSA levels but instead is based on readings from tissue near the prostate.

Thus, according to one aspect, the present invention provides a method of diagnosing prostate cancer in a subject, which method comprises determining the concentration of a relevant biochemical marker in the periprostatic tissue of said subject.

There is a widely used system of staging prostate cancer called the TNM System. This system describes the extent of the primary tumour (T stage), the degree of spread to regional lymph nodes (N stage) and the degree of distant spread, or metastasis (M stage). In the T stage 4 separate stages are defined:
T1 - tumour is impalpable during a digital rectal exam (DRE) and it is non-visualized by ultra-sound imaging, however cancer cells can be found in biopsy samples
T2 - tumour is palpable during DRE, locally confined within the prostate gland i.e. within the capsule
T3 - tumour has extended through the prostatic capsule; locally extensive to the seminal vesicles but no other organs are affected
T4 - tumour has invaded neighbouring organs other than the seminal vesicles

Prostate Specific Antigen (PSA) is a glycoprotein enzyme having a molecular weight of about 30 kDa, the protein is exclusively produced by prostate epithelium cells. The PSA concentration in the seminal plasma is a few g/l while the normal systemic blood PSA concentration is a few µg/l, i.e. the normal concentration ratio between the inside and outside of the gland is in the range of 10⁴- 10⁶; this ratio is reduced in the case of a prostate cancer penetrating the gland capsule and invading the surrounding organs.

In the normal situation the PSA producing cells are only found inside the prostate gland. The presence of such cells in periprostatic tissue (i.e. stages T3 and T4) is an indicator of locally extensive or invasive cancer progression. If the tumour is infiltrating neighbouring tissues, the PSA concentration of these tissues is increased and the concentration gradient across the capsule, relative to both the periprostatic tissue and the serum, is less steep compared to the normal healthy situation.

In general, a PSA concentration ≥ 1mg/l measured at a specific position is an indication of PSA producing cells at this position. Thus, for example, if this position is outside the prostatic capsule, e.g. in the periprostatic tissue, the diagnosis of a T3 or T4 stage tumour is indicated.

As an alternative to or in addition to an absolute value indicating a T3 or T4 tumour, it may be helpful to consider the ratio of the concentration of the marker within the periprostatic tissue as compared to the serum concentration. In general, a T3 or T4 tumour is suggested by a ratio of the PSA concentration within the periprostatic tissue/serum of greater than 100. Thus in a preferred embodiment, the methods of the present invention further comprise determination of the concentration of the relevant biochemical marker in the serum of the patient.

Presence of a marker such as PSA in the local or other lymph nodes is also indicative of a tumour which has extended beyond the prostatic capsule.

The present invention provides a method of identifying a prostate tumour which has breached the prostatic capsule (i.e. a T3 or T4 tumour), which method comprises determining the concentration of a relevant biochemical marker in the periprostatic tissue or lymph nodes of said subject.

Alternatively viewed the invention provides a method of categorising a prostate tumour in a subject, which method comprises determining the concentration of a relevant biochemical marker in the periprostatic tissue or lymph nodes of said subject.

The 'periprostatic tissue' is the tissue surrounding the prostate capsule and includes the Denonvilliers' fascia, lateral pelvic fascia and associated neurovascular bundle, the anastomosis region, bladder neck, the region around the prostate apex, the seminal vesicles and pelvic glandulas and lymph nodes on the pelvic wall. Depending on the patient, this region often incorporates significant amounts of fatty tissue.

The lymph nodes which may be assayed will preferably be localised near the prostate but the lymph circulation means the marker may be found further from the prostate; nevertheless the preferred lymph nodes to be assayed are the prostate sentinel lymph nodes.

More particularly the invention provides a method of diagnosing locally extended prostate cancer, i.e. diagnosing a tumour which has extended beyond the prostate capsule. The present invention provides a method of diagnosing a locally extended prostate cancer due to the elevated concentration of certain biochemical markers in the periprostatic tissue or lymph nodes when the tumour has broken through the prostate capsule.
Thus the invention also provides a method of diagnosing a T3 or T4 stage tumour and methods of differentiating between T1 and T2 tumours (and no tumour) on the one hand and T3 and T4 tumours on the other. A method of tumour categorisation is therefore provided, the technique enabling a T3 or T4 stage tumour to be identified. Such a categorisation may be performed when the existence of a prostate cancer is known or suspected on in subjects where there is no previous data to suggest a prostate cancer.

Preferably one or more measurements is taken at a distance of at or around 1 mm outside of the prostatic capsule. Ultrasound imaging can be used to determine these distances. In general the most interesting measurement regions are in the upper part of the prostate at the left and right posterolateral and the posterior positions since most prostate tumours develop from stage T2 to stage T3 in these regions.

In preferred embodiments, as well as determining the concentration of a relevant biochemical marker in the periprostatic tissue, the concentration of the same marker within the prostate itself, i.e. inside the prostate capsule, is also determined. The two measurements are made at the same or substantially the same time, e.g. as part of the same invasive procedure, either by analysis ex vivo of two separate samples or preferably by real time in situ measurements, as described in more detail below.

In a preferred embodiment, 2 or more, preferably 3 or 4 or more e.g. 5 or 6 different measurements are taken which extend from within the prostate, across the capsule and into the periprostatic tissue. These different sample points will typically be in a line, or substantially in a line, which crosses the capsule. In the case of a T3 stage tumour, the capsule may be breached but the tumour does not actually yet extend into the periprostatic tissue. In such a case, a series of sample points on a line through this tumour would show a gradual decrease in [marker] from inside to outside. A series of sample points which did not cross a tumour would have a distinctive sharp drop in [marker] between the point on the inside closest to the capsule and the first point outside the capsule in the periprostatic tissue.

The concentration of the marker will preferably be measured by sampling the extracellular (interstitial) fluids, preferably *in situ,* e.g. using the probes described herein or ex *vivo* on extracted samples, e.g. using immunoassay techniques. Where whole tissue concentrations are measured then both intracellular and extracellular fluids will be included in the assay. However, even when the extracellular fluids are the target sample, the intracellular fluids may contribute to the measurement depending on the sampling technique or probe used and the extent of tissue damage. Preferably the amount of intracellular fluid in the sample is minimised, e.g. by using weak aspiration/suction to extract fluid from the biopsy core.

The extracellular fluid can be extracted for analysis via a cytology insertion needle or via microdialysis or ultrafiltration techniques. The probe can be used in situ either by insertion directly into the insertion needle, or, more preferably, by insertion into a tube which also can be used for extracting cytology samples in parallel.

Where the measurement is taken in the lymph nodes then typically it is the concentration of marker within the lymph plasma which is measured.

The most studied and preferred biochemical marker is PSA (hK3) but other suitable markers include human glandular Kallikren 2 (hK2). The concentration of hK2 inside the gland, e.g. in the seminal plasma is typically 0.5 mg/l and in serum is typically 0.1 µg/l. Therefore the concentration ratio is normally in the range of 10³-10⁴. Preferably the [hK2]periprostatic / [hK2]serum ratio is used. This ratio is preferably greater than 100.

'Relevant biochemical markers' are those which are produced by the prostate and/or by a prostate cancer and whose concentration in the periprostatic tissue shows a significant increase when a prostate cancer has extended through the prostatic capsule. Typically, suitable markers will exhibit a significant drop in their concentration ratio between the inside and outside of the prostate when a prostate cancer has extended through the prostatic capsule.

In preferred embodiments of the methods of the present invention the concentrations of the relevant biochemical markers are determined in situ, i.e. a sample is not removed from the subject for analysis. Concentrations are determined by an invasive measurement device which incorporates a sensor for one or more relevant biochemical markers.

Suitable sensors may be based on the hydrogel fiber optic sensors described in GB 2 385 915, or on the surface plasmon technique (e.g. based on devices disclosed by Homota et al. in Sensors and Actuators B54 (1999), 3-15) or on immunoassays. The sensitivity of the hydrogel may be based on enzyme/substrate or antibody/antigen interactions. Preferably the sensor will incorporate an antibody to the marker antigen or where the marker is an enzyme, a substrate for that enzyme.

Suitable embodiments and variants of the sensors described in GB 2385915 may comprise a polymer gel, such a gel may have additional cross-links formed between antibodies and antigens immobilised on the polymer chains. This antigen could either be a native antigen (i.e. natural protein) or a peptide sequence similar to a linear epitope on the native antigen. Such a hydrogel will swell in the presence of a free antigen because antigen-antibody cross-linked binding can be disassociated by exchange of the immobilised antigen for the free antigen. In the absence of the free antigen, the gel will shrink.

In some embodiments, the gel comprises a biomolecular recognition component linked to the polymer chain. Such linking may be direct or indirect through one or more linking molecules. Examples of such biomolecular recognition components include enzymes, antibodies, antigens and aptamers. The hydrogel volume changes when a chemical species binds to the recognition component and changes the hydrophobicity of the gel. Alternatively, the hydrogel refractive index may change when analyte molecules bind to the recognition component. In preferred embodiments enzymatic substrates are used as the biomolecular recognition component. The enzymatic substrates are preferably formed from short peptides which are selected such that they respond to at least one target enzyme. When a target enzyme is present in the sample it is able to diffuse into the gel and cut the peptides forming the cross-links, causing a change in volume or refractive index of the gel.

Such a gel containing sensor is described in the Examples. In the present invention the enzymatic substrate is preferably a PSA substrate, and preferably a peptide, e.g. a peptide derived from semenogelin I or II.

Ionic hydrogels are preferred. Ionic hydrogels are formed by immobilizing ionizable groups on the polymer chain. Preferably, the ionic hydrogel is formed by adding an ionizable monomer to a pre-gel solution comprising another monomer and a cross-linker. For example, ionic monomers such as acrylic acid (anionic) or dimethylaminopropylacrylamide (cationic) may be added to a pre-gel solution containing acrylamide and the cross-linker bisacrylamide. The degree of ionization of the hydrogel and the concentration of counter ions can be varied by altering the pH and the ionic strength of the solution respectively so as to change the equilibrium swelling of the hydrogel. This ionic strength of the solution may be varied by, for example, adding salts to the solution.

These sensors allow measurements to be taken quickly (in 'real time'), i.e. there is effectively no delay or a minimal delay between the in situ "sampling" and the presentation of the information on concentration to the tester. This delay will typically be no more than 5 minutes, preferably less than 4 minutes, more preferably about 3 minutes or less. Classical "sampling" is not performed with the sensors used in the method of the invention but there is in effect a reading taken of a sample of extracellular fluid which comes into contact with the sensor.

An ex *vivo* method can still provide information in a short period of time, e.g. if a microdialysis or an ultrafiltration probe is used.

The durations mentioned above in the context of real time measurements are exclusive of the time it may take to get the sensor(s) into position to take the measurements, e.g. for insertion of the needle.

The prostate needle biopsy is the present state-of-the-art reference diagnostic procedure if prostate cancer is suspected. A biopsy is a surgical procedure to remove cells or tissue from the body in order to look at them under a microscope to check for signs of disease. The microscopy examination is performed by a pathologist. Current practice is to remove 6-8 tissue samples from the prostate gland under Trans Rectal Ultra-Sound (TRUS) image guidance. The tissue samples are harvested with a specialized biopsy needle, being hand held or integrated with the TRUS imaging probe. The TRUS image guides the tissue biopsy procedure as an ultrasound video image created from tissue density variations.

Knowledge of local PSA concentrations at selected sample points obtained from in situ real time measurements taken together with the tissue biopsy samples improves the diagnostic quality of the biopsy procedure. This way the tissue samples will be harvested at positions determined from the combination of relevant information from physical (tissue density) and chemical (e.g. PSA concentration) properties of the suspected regions.

Typically the 20 minutes TRUS biopsy procedure is carried out in the office of the urologist and the report from the pathology examination of the samples is ready some days later. This time delay between tissue sample harvesting and pathological evaluation is a limitation of the prior art biopsy procedure as a tool promoting precise, cost effective and real time staging in the diagnosis of prostate cancer.

During examination, when a surgeon is selecting regions for biopsy, real time PSA values may be used to indicate areas from which a biopsy should be taken.

Obtaining information in real time, as opposed to following biopsy analysis, is also advantageous during open surgery where sensing increased PSA levels, indicative of PSA producing cells, may be used as a guide for the surgeon in the determination of the extent of the tumour.

Thus, the diagnostic methods may be performed pre- (or in the absence of) or during peroperative staging of prostate cancer. Thus a sensor may be used to measure the concentration of PSA in tissues during:
1) Preoperative staging of the prostate cancer tumour, e.g. as a tool for choosing a suitable therapy, and
2) Peroperative staging under radical prostatectomy, e.g. as a tool for the surgeon in defining eventual extracapsular tumour infiltration in the apex, bladder neck, neurovascular bundle region, vesicula seminalis and suspect lymph nodes, and
3) Diagnostic investigation for demonstration of metastatic tumours, e.g. in lymph nodes.

Suitable measurement procedures include:
1) For preoperative staging for choosing therapy:
   - PSA is measured in tumour tissue before biopsy
   - PSA is measured in periprostatic tissue
   - PSA is measured in vesicula seminalis
2) For peroperative staging during radical prostatectomy:
   - PSA is measured in periprostatic tissue in the tumour region
   - PSA is measured in suspect lymph nodes
   - PSA is measured in bladder neck and apex region
      A) For follow-up of patients having received curative treatment of localized prostate cancer:
         - Local ultrasound + PSA measurement in the anastomosis region
         - TRUS + PSA measurement in suspect pelvic glandulas on the pelvic wall
      B) For follow-up after curative radiation (X-ray or gamma) treatment
         - TRUS + PSA measurement in the remaining tissue of the prostate, possibly including in periprostatic tissue.

A sensor in the form of a needle probe can be inserted from transrectal or perineal position under local anaesthesia in a similar manner as a biopsy needle. The probe is preferably inserted under image guidance (ultrasound, MRI). Imaging is helpful for three reasons: 1) for monitoring the position of the sensor, 2) for identification of suspect tissues, and 3) for recording of measurement positions and related PSA concentration measurements.

In each measurement position the sensor is exposed to the tissue fluids, this is conveniently performed by a manual operation of the probe and is allowed to register a PSA measurement, e.g. for 1-4 minutes, preferably about 3 minutes, before moving to the next position or termination.

The sensor, e.g. the needle probe, may comprise means for extracting a sample of extracellular or tissue fluid, these fluids can be subjected to *ex vivo* analysis. Moreover, when the measurements are not performed in situ, apparatus for determination of the concentration of a biochemical marker within the periprostatic tissue may be provided which has means for ex *vivo* analysis of extracellular or tissue fluids, e.g. by microdialysis or ultrafiltration.

The sensor, e.g. the probe, may be used to measure marker concentration in interstitial fluid in a sample, e.g. a biopsy, which is ex vivo.

As discussed above, the methods of the invention preferably involve invasive measurements using sensors, image guidance (e.g. TRUS) is preferably used to determine the positions in the periprostatic tissue or the prostate at which the readings are taken. When more than one measurement is made this may be via a single sensor or using multiple sensors. Where a tissue biopsy needle is used this may also conveniently incorporate the sensor for performing the measurements in accordance with the present invention.

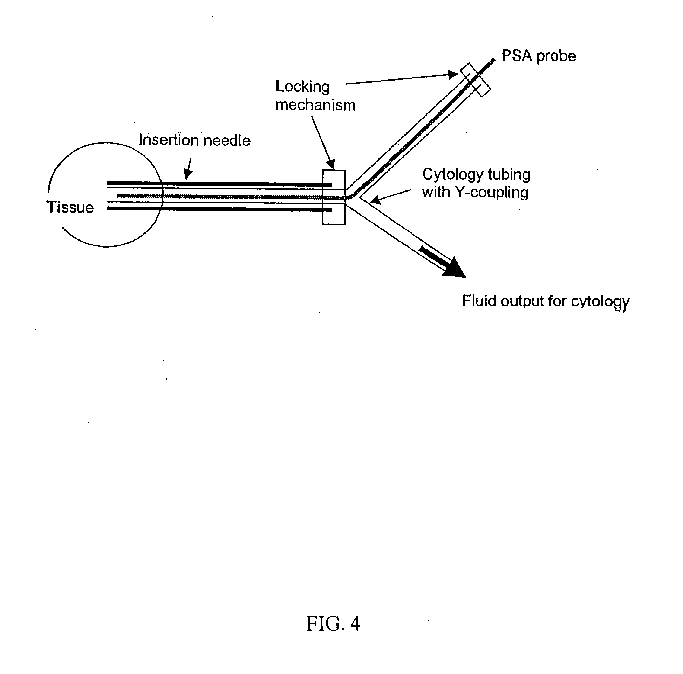

In a further aspect, the present invention provides apparatus for in situ determination of a relevant biochemical marker within the periprostatic tissue of a subject. The apparatus incorporates a sensor for said biochemical marker, suitable sensors are discussed above. The apparatus may conveniently be in the form of a needle, e.g. a needle adapted for the taking of biopsies. A needle like form to the probe means it is convenient for both TRUS guided invasive diagnosis as well as during radical prostatectomy. Such an apparatus is shown schematically in Fig. 4.

The present invention is based on an understanding that certain biochemical markers produced by cancer cells are usually produced at high concentrations within the prostate gland but when a cancer has extended beyond the prostatic capsule, that marker is found in higher than normal concentrations outside the gland. Thus, in a further embodiment the present invention provides a method of diagnosing prostate cancer in a subject, and a method of identifying a prostate tumour which has extended beyond the prostatic capsule of a subject, which method comprises determining the concentration of a relevant biochemical marker in the non-prostate tissue of said subject. 'Non-prostate tissue' excludes the prostate capsule and does not include blood serum. Preferred non-prostate tissue, as discussed above, includes the periprostatic tissue but also lymph nodes (not just those on the pelvic wall) and anastomosis region. Preferred embodiments and suitable sampling methods and apparatus discussed above in connection with the periprostatic tissue apply mutatis mutandis to these other areas.

The invention is further described in the following non-limiting Examples and with reference to:

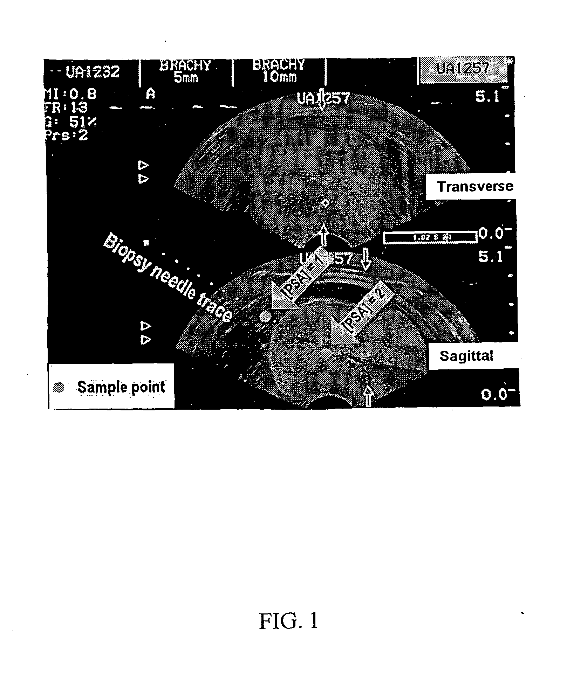

Fig. 1 which is a photograph of a TRUS sagittal image of part of the prostate. In Fig 1 the line of a putative biopsy needle trace is marked as are two sites at which the PSA concentration may be determined. [PSA] 1 is from the periprostatic tissue and [PSA] 2 from within the prostatic capsule.

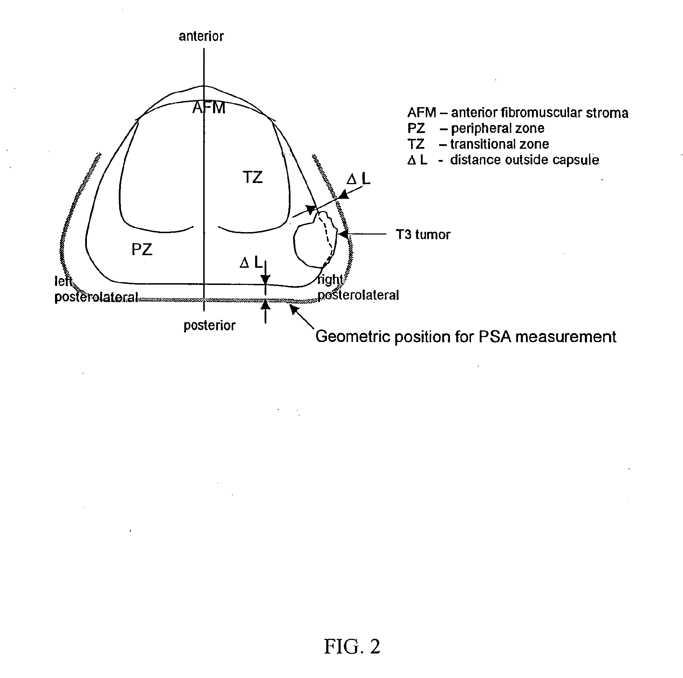

Fig. 2 is a schematic cross section of the prostate glandula with stage T3 tumour indicated at the right posterolateral position. The line at ΔL surrounding part of the capsule shows the point at which PSA in the periprostatic tissue is measured.

The reference PSA concentration is the systemic concentration [PSA]₀. Measurements taken at the geometric position ΔL is termed [PSA]_{ΔL}. The criterion for a likely extensive tumour (T3) region is set at the ratio: [PSA]_{ΔL}/[PSA]₀ > 100.

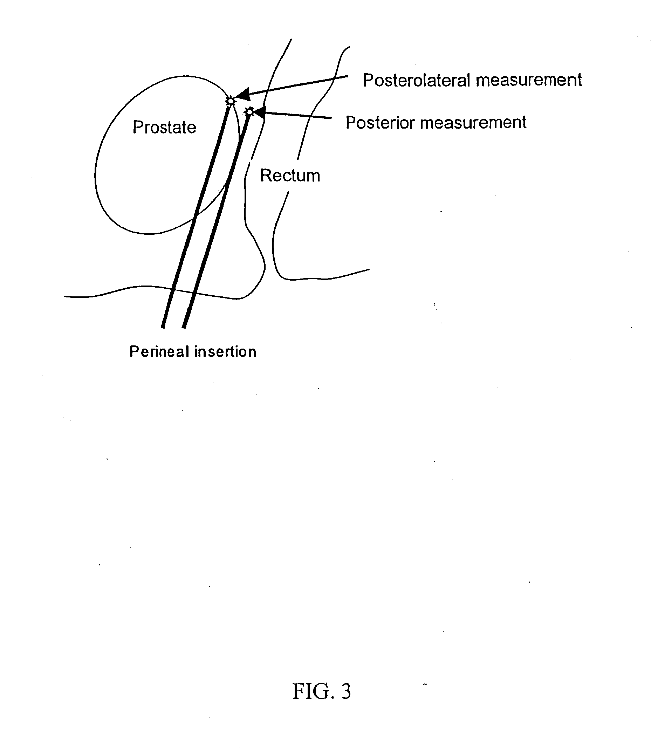

Fig. 3 is a sketch of a longitudinal view of the prostate showing perineal insertion of measurement probes indicated at posterior and posterolateral positions.
Fig. 4 is a sketch showing suitable apparatus for in situ marker measurement and cytology sampling.
Fig. 5 is a graph showing Tissue-PSA concentrations for tumour, normal prostate and periprostatic tissue samples.
Fig. 6 is a graph showing Tissue-PSA concentration of extracapsular (periprostatic) tissue samples vs pathological T-stage values.

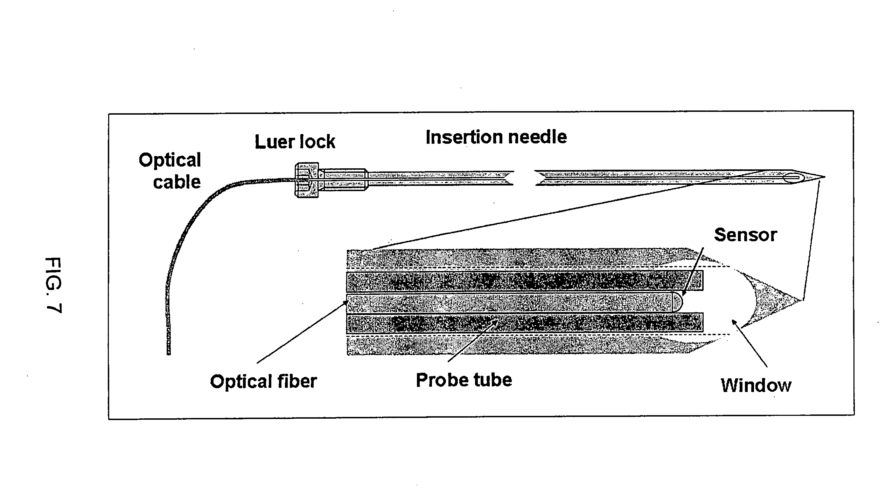

Fig. 7 is a drawing of probe suitable for performing the methods of the invention.

### Example 1

Biopsy cores from 18 resected radical prostatectomy specimens were homogenized and characterized with respect to tissue-PSA concentration using an immunoassay technique. The Immunoassay was a Perkin Elmer assay, DELFIA^{®}, this is a time-resolved fluorometric assay including an enhancement step. PSA antibodies (mAb E86 labelled with Europium (Eu³⁺) are used).

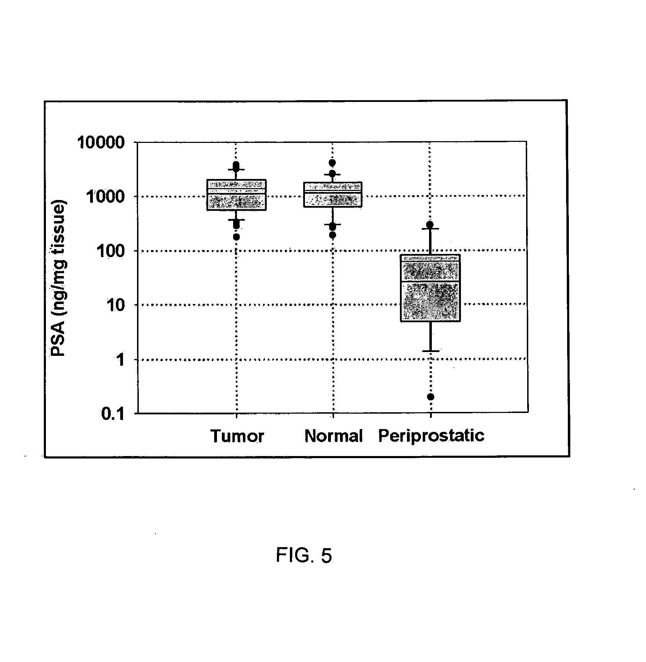

Periprostatic tissue samples (including adipose tissue) were also dissected from the same specimens and characterized with respect to tissue-PSA concentration. Figure 5 shows the tissue-PSA concentrations measured in prostate tumor, normal prostate, and periprostatic tissue. The boundary of the box indicates the 25th and 75th percentiles, the lines within the box mark the mean and median. The error bars indicate the 90th and 10th percentiles. The periprostatic tissue-PSA concentrations are typically one to three orders of magnitude lower than the prostatic tissue-PSA concentrations.

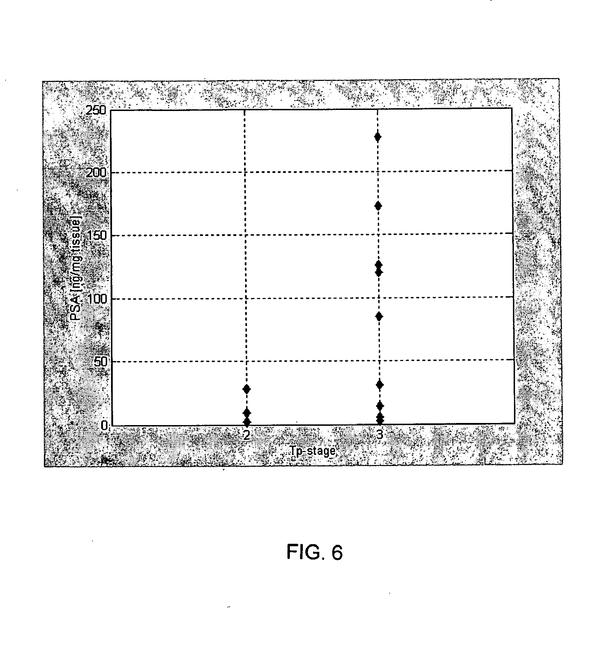

After the tissue samples were taken, the specimens were assessed and staged by a pathologist. Figure 6 shows tissue-PSA concentrations of extracapsular tissue samples vs pathological T-stage values for all 12 specimens where both extracapsular tissue samples and pathological stage value were available. The extracapsular tissue-PSA values from the Tp3 specimens are clearly higher than the PSA values from the Tp2 specimens.

To estimate the corresponding PSA concentration in interstitial fluid (ISF) we measured the PSA concentration in tissue fluid obtained by placing prostate tissue samples in centrifuge tubes provided with a basket of nylon mesh with pore size 15*15 µm² and spun at 2000 rpm for 10 min. The samples included prostate gland tissue as well as periprostatic tissue. A PSA sensitive probe response was recorded before the ISF PSA concentrations were measured using an immunoassay.

A PSA sensitive probe as described herein and in GB 2385915 and incorporating a polyacrylamide hydrogel crosslinked with bisacrylamide and PSA specific substrates was used. The substrate is a peptide fragment or derivative of semenogelin I and II, these are known substrates for PSA (Malm et al. The Prostate [2000] vol. 45, pp 132-139).

The probe response was measured in interstitial fluid extracted using established protocols (Wiig et al. American Journal of Physiology Heart and Circulatory Physiology [2003], vol. 284, pp H416-H424).

The PSA concentration in interstitial fluid averages 625 µg/ml, determined by immunoassay. The tissue-PSA concentration (inter- and intracellular) in the same normal and tumor tissue as measured using immunoassay averaged 1360 ng/mg tissue (Figure 5). This provides a calibration factor to convert from PSA amount per unit weight of tissue to ISF [PSA]. Using the same calibration factor for the extracapsular tissue samples included in Figure 6, we find that for pathological T3 stage the extracapsular interstitial PSA concentration can be in the range from 1 to 100 µg/ml.

It was found that the PSA sensitive probe response scales linearly with ISF PSA concentrations determined by immunoassay. One calibration factor for the probe can be used for all tissue types.

### Example 2

A PSA sensitive probe was inserted into intracapsular and extracapsular (periprostatic) tissue samples from resected radical prostatectomy specimens. A small volume, 12% (volume to tissue weight), of physiological saline buffer solution was added to the sample container to prevent sample desiccation. The probes were left in position for about 5 min before the local interstitial fluid PSA concentration responses were presented. A total of 25 probes were tested in tissue samples from three different specimens. Homogenized tissue samples from the same specimens, as well as from a large number of biopsy cores (74 cores from 18 specimens) were characterized with respect to PSA concentration using immunoassay. A number of the PSA characterized homogenizes tissue samples were used to validate the PSA probe responses.

The PSA responses from probe insertions into prostate cancer tissue samples corresponded to interstitial fluid PSA concentrations up to 150 µg/ml. The corresponding PSA concentration measured in the homogenized tissue samples was 260 ng/mg tissue. In extracapsular adipose tissue samples the probe response corresponded to 1.5 µg/ml. The corresponding PSA concentration measured in homogenized adipose tissue samples was 6 ng/mg tissue. The PSA concentration of the homogenized tissue samples averaged 1361 ng/mg tissue for intracapsular biopsy cores, whereas extracapsular tissue samples, including suspect extracapsular extension samples, averaged 68 ng/mg tissue, i.e. comparable to the interstitial fluid PSA concentrations estimated using PSA sensitive probes.

We have shown that the near real time PSA probe is sensitive enough to differentiate between prostate cancer tissue and periprostatic tissue. For diagnostic and staging the probe can be used to identify extraprostatic tumor extension by positioning the probe into suspect periprostatic tissue using an insertion needle under TRUS guiding.

### Example 3

Perineal TRUS guided needle biopsies (18G) of periprostatic tissue of two patients were taken. Immediately following the biopsy procedure, tissue fluid (100 nL) was extracted from the biopsy cores using the PSA sensitive probe, and the PSA response were recorded. The biopsy cores were transferred to tubes with buffer and frozen. The samples were later diluted (500 uL) and homogenized and characterised with respect to tissue-PSA concentration using immunoassay. The PSA probe results showed good correlation with immunoassay results. More than 94% of the ISF PSA is free-PSA. The probe results can be used to differentiate between PSA producing tissue and normal periprostatic tissue.

## Claims

1. A method of diagnosing prostate cancer in a subject, which method comprises determining the concentration of a relevant biochemical marker in the periprostatic tissue or lymph nodes of said subject.

2. A method of categorising a prostate tumour in a subject, which method comprises determining the concentration of a relevant biochemical marker in the periprostatic tissue or lymph nodes of said subject.

3. A method of identifying a prostate tumour which has extended beyond the prostatic capsule of a subject, which method comprises determining the concentration of a relevant biochemical marker in the periprostatic tissue or lymph nodes of said subject.

4. A method as claimed in any preceding claim wherein the concentration of said biochemical marker within the serum of said patient is also determined.

5. A method as claimed in any preceding claim wherein the concentration of said biochemical marker within the prostate gland of said patient is also determined.

6. A method as claimed in any preceding claim wherein 2 or more determinations of the marker concentration at different locations within the periprostatic tissue are made.

7. A method as claimed in any preceding claim wherein the marker concentration within the periprostatic tissue is determined at a position about 1 mm outside the prostatic capsule.

8. A method as claimed in any preceding claim wherein the determinations are performed in situ.

9. A method as claimed in a any preceding claim wherein the determinations are performed in real time.

10. A method as claimed in a any preceding claim wherein the marker is PSA or hK2

11. A method as claimed in any preceding claim wherein the marker is PSA.

12. A method as claimed in any preceding claim wherein a sensor is brought into contact with the periprostatic tissue or lymph nodes *in vivo* in order to determine the marker concentration.

13. A method as claimed in claim 12 wherein the sensor is incorporated into a needle probe.

14. A method as claimed in claim 12 or 13 wherein the sensor incorporates a hydrogel.

15. A method as claimed in any one of claims 12 to 14 wherein the sensor comprises an antibody for the marker.

16. A method as claimed in any one of claims 12 to 14 wherein the sensor comprises a substrate for the catalytic activity of the marker.

17. A method as claimed in any one of claims 13 to 16 wherein the needle probe comprises means for extracting a sample of said periprostatic tissue.

18. A method as claimed in any one of claims 13 to 17 wherein the needle probe comprises means for extracting a sample of extracellular fluid or tissue fluid.

19. Apparatus for *in situ* determination of the concentration of a biochemical marker within the periprostatic tissue or lymph nodes of a subject.

20. Apparatus as claimed in claim 19 comprising a sensor as defined in any one of claims 14 to 16.

21. Apparatus as claimed in claim 19 or 20 in the form of a needle probe.

22. Apparatus as claimed in any one of claims 19 to 21 comprising means for extracting a sample of said periprostatic tissue, lymph nodes or of extracellular or tissue fluid.

23. Apparatus for ex *vivo* determination of the concentration of a biochemical marker within the periprostatic tissue or lymph nodes of a subject.

24. Apparatus as claimed in claim 23 which comprises means for determination of the marker concentration by microdialysis or ultrafiltration.
